# EUROPEAN PATENT SPECIFICATION

(11) **EP 3 139 863 B1**
(45) Date of publication and mention of the grant of the patent: **22.01.2020**
(21) Application number: 15722988.1
(22) Date of filing: 06.05.2015
(51) Int. Cl.: A61F 2/24

(54) **PROSTHETIC VALVE AND METHOD FOR MAKING SAME**
KLAPPENPROTHESE UND HERSTELLUNGSVERFAHREN DAFÜR
VALVULE PROTHETIQUE ET SON PROCEDE DE PRODUCTION

(30) Priority: 06.05.2014 EP 14167271; 06.05.2014 EP 14167270; 06.05.2014 EP 14167269; 06.05.2014 EP 14167272
(43) Date of publication of application: 15.03.2017
(73) Proprietor: DSM IP Assets B.V., 6411 TE Heerlen (NL)
(72) Inventor: GRUNDEMAN, Paul Frederik, NL-3584 CX Utrecht (NL); KLUIN, Jolanda, NL-3584 CX Utrecht (NL); BOON-CEELEN, Karlien Kristel, NL-6100 AA Echt (NL); KÖNIG, Thomas, NL-3584 CS Utrecht (NL)
(74) Representative: DSM Intellectual Property
(86) International application number: PCT/EP2015/059985
(87) International publication number: WO 2015/169869

(56) References cited:
- WO-A1-00/62714
- US-A- 3 526 906
- US-A- 3 859 668
- US-A1- 2008 200 977
- US-B2- 7 465 316

## Description

### General field of the invention

The invention relates a method of making a prosthetic valve and more specifically a two- or three-leaflet prosthetic heart valve.

### Background

A typical natural valve of a mammal is the aortic valve, one of the four heart valves. The aortic valve comprises three leaflets, also called cusps, attached to the aortic root that serves as a supporting element for these leaflets. Each of the three leaflets of the aortic valve has a free margin and a margin where it is attached in semilunar fashion to the aortic root. When the valve opens, the leaflets fall back into their sinuses without the potential of occluding any coronary orifice. The hingelines of adjacent leaflets meet at the level of the sinutubular junction, forming at least part of the commissures. The body of a leaflet is pliable, extendable and thin to provide the required flexibility, although its thickness is not uniform. The leaflet is slightly thicker towards its free margin. On its ventricular surface is the zone of apposition, known as the lunule, occupying the full width along the free margin and spanning approximately one-third of the depth of the leaflet. This is where the leaflet meets the adjacent leaflets during valvular closure. With the valve in closed position, the margins of the lunules meet together, separating blood in the left ventricular cavity of the heart from blood in the aorta. For a valve of this type, or a corresponding type, highest mechanical stresses during opening and closing occur at the commissures and, to a lesser extent, at the free margin of the leaflets.

Prosthetic valves are implanted in the human or animal body and may for instance be used as a passive, one direction prosthetic valve within or nearby blood vessels. They can be completely preformed and implanted as such, or formed in situ using the artificial and/or natural parts needed to form a functional prosthetic valve. A suitable prosthetic valve needs to open and close readily in response to differential pressure on either side of the valve, cause no or only little non-physiological turbulence in the blood flow, and avoid too much regurgitation. Cardiovascular products, such as heart valve prostheses, are thus subject to high requirements with respect to loading conditions, both in magnitude as in number of cycles. Typically, heart valve leaflets may undergo over a billion load cycles in their lifetime. Durability of prosthetic valves, especially of moving leaflets, is therefore an important requirement.

Any prosthetic valve should be able to resist the actual mechanical load on the commissures and leaflet free margin during valvular operation and preferably, maintain to resist such cyclical load during many years. For this, not only initial strength is an important parameter but also reducing the chances of (non-apparent) production anomalies in making the valve.

Today, valves used in valve surgery typically are bioprosthetic valves having leaflets made from biological tissue, often chemically treated bovine pericardium. This is an elastic material that performs relatively well and is able to mimic the natural valve. However, early failure is often encountered, and is believed to be associated with high stresses on the leaflet material upon continuous stretching and retracting under pulsatile load. Various methods have been proposed as alternatives for making leaflets of prosthetic valves wherein synthetic materials and alternative designs are used.

A valve prosthesis made using synthetic fibers is for example described in NL1008349. This valve comprises a supporting element carrying a number of leaflets, which have been made by winding reinforcing fibers onto a mandrel in specific directions corresponding to the occurring stresses in the leaflets. Since the fibers have to be positioned according to the maximum stress lines, this valve prosthesis is difficult to make and uses many wound layers to accommodate stresses, whereby mass is added.

Similarly, US6726715 describes a leaflet for a heart valve comprising a flexible sheet having stress-relieving fibrous elements aligned with predetermined stress lines in the leaflet during valve operation. Sheet material is typically PTFE or PVF, with high-strength/high-modulus fibers as reinforcing elements. Fibers such as carbon, aramid, or polyethylene fibers like Dyneema® UHMWPE fibers may be used.

WO2010/020660 describes a prosthetic valve made from a uniform hollow braid made from polyolefin fibers. The hollow braid is shaped to form a valve by pulling it over a mould, comprising a tubular part and a star-shaped part. By subsequently applying heat and pressure, the hollow braid takes the shape of the mould and different sections are created. Around the tubular part of the mould the braid forms into a section that corresponds to a supporting element of the valve, whereas a star shaped part of the mould provides a section that corresponds to multiple valve leaflets. Before removing the valve from the mould, the front and back sides of the valve prosthesis are edge trimmed. To prevent disruption of the trimmed edge, the edge may be heat treated to melt fuse the yarns to each other, provided with a stitching, or otherwise treated to make the edge mechanically stable.

WO 2004/032987 concerns a medical device having at least three layers of polymeric components arranged in a sandwich construction, wherein the polymeric component of the middle layer has a shorter chain length than the other polymeric components. A heart valve is mentioned as a possible application of the sandwich construction.

Heim et al. (Materials and Manufacturing Processes, 26: 1303-1309, 2011) disclose a method wherein artificial leaflets are made from woven polyester yarns by thermally shaping the woven textile on a mold into a three-cusp geometry; showing that woven polyester could be suited to form a valve prosthesis. Polyester yarn has stretching properties such that the woven textile is able to mimic the natural elastic stretching of a human valve (about 15% of elongation), due to its typical elongation at break of about 14-17%. In order to obtain a valve with good contact between leaflets in closed position and to limit stresses during working cycles, the authors teach to shape the leaflets such that there is a fairly large inherent opening in the center of the valve, whereas under cardiac pulsatile load adequate coaptation is created over the length of the free margin of the leaflets to prevent or at least minimize regurgitation.

In US2005/0137681 a venous valve with a tubular frame and a cover is disclosed, which cover includes surfaces defining a reversibly sealable opening and thus acting as leaflets. The leaflets can have various sizes and shapes, including arcuate edges, curved surfaces, a concave structure, or include a curved support structure to efficiently close the valve and restrict retrograde fluid flow. Leaflets may be made of biologic or synthetic fluid-impermeable material, including ePTFE, PET, urethane and polyethylene.

WO2000/62714 discloses a heart valve prosthesis including a one-piece moulded body with a plurality of leaflets, made from a silicone or polyurethane. In the neutral or rest position, the leaflets' free margins converge to form a non-uniform gap between them. The leaflets have a scallop in their free margins, proving sufficient material at the center to seal against reversed fluid flow with minimum coaptation.

US2004/176658 relates to a medical support net adapted to be placed around an organ; for example a cardiac support net, which is made as a multilayered fabric by a warp knitting technique, preferably from multifilament polyester yarn.

US4191218 discloses woven fabrics for use in vascular prostheses and heart valves, which fabrics are woven from multi-filament (polyester) yarns comprising filaments of about 10 µm diameter, and which fabrics are heat shrunk to result in open interstitial space of 20-40 µm and elongation in at least one direction of at least 10%. The fabrics preferably have a woven selvedge, which forms the free margin of a heart valve leaflet.

In US2005/177227 a method of making a cardiac valve prosthesis is disclosed, wherein a textile membrane, preferably made from polyester or PTFE, is shaped to form leaflets; for example by cutting out segments and using a shaped member reproducing the geometry of a cardiac valve in closed artery position, followed by thermofixation. It is indicated that a leaflet preferably has a woven or knitted free edge to avoid raveling.

US2008/275540 describes methods for making a tubular network stent for use in an artificial heart valve, more specifically making of a two- or multilayer stent by interweaving at least one elastic metal line is disclosed.

US4035849 discloses a heart valve prosthesis comprising leaflets made from natural tissue and a stent, wherein the stent comprises an annular frame with three ventricular struts, the exterior surface of which is covered with a fabric. The fabric is non-absorbant, can be single- or multi-layered, and is typically made from polyester or from PTFE.

US2012/0172978 describes a prosthetic valve comprising leaflets made from an isotropic filter screen material that has uniform pores of 15-60 µm and a thickness of 10-100 µm, and which material is woven from e.g. polyester or polypropylene monofilaments. In response to a closed flow pressure the leaflets can be pushed together to engage at the outflow edge. Methods of making such valve comprise steps of forming separately leaflets from a single layer of screen material, coupling them together along an attachment line, and optionally coupling to a sewing ring or stent. The attachment line forms a commissure, optionally in combination with connected tabs extending from the ends of the free margin of leaflets at the outflow edge. Typically leaflets are cut from the screen material in such way that the edges of a finished leaflet do not substantially have any extending fibers. Document WO-A-00/62714 discloses method of making a one piece leaflet assembly.

Still, there is a continuing need for a method of making implantable prosthetic valves having adequate properties for replacing a natural valve, especially for prosthetic valves showing very good durability.

### Summary

The present invention provides a method of making a prosthetic valve (400) that can take a first form wherein the valve is open and a second form wherein the valve is closed, the valve comprising a leaflet assembly having at least two leaflets (3) attached to a supporting element (2), the leaflets having a free margin (5) that can move between a first position wherein the valve takes the first form and a second position wherein the valve takes the second form, the method comprising:
- providing a single piece of fabric, made by weaving warp and fill threads into a seamless tubular woven fabric having at least one stabilized edge, and
- forming the fabric into a leaflet assembly having an inner layer forming the leaflets with the stabilized edge forming the free margin, and an outer layer forming the supporting element.

In this method a single piece of a seamless tubular woven fabric is used for making a tubular leaflet assembly comprising at least two leaflets and a supporting element, with the free margin of the leaflets being formed from a stabilized edge of the woven fabric. Prior methods typically make a leaflet assembly from one or more pieces of material, that are assembled and connected to each other, thus typically creating seams. Such tubular fabric can be made with a weaving technique making two or more connected layers, commonly referred to as double weaving, resulting in a flat or flattened woven tubular fabric. The tubular fabric thus made may have one tubular layer defining one longitudinal tube or channel, but can also be a multilayer construction having multiple tubes or channels. Alternatively, a tubular fabric having multiple tubes or channels can also be woven by using a warp beam having e.g. ring-shaped design reflecting the desired tubular cross-sectional design. An end or edge of a tubular fabric, parallel to fill threads and perpendicular to warp thread direction, may be stabilized against fraying after weaving the fabric, or may be woven as a selvedge. Before or during attaching the leaflet assembly to an optional stent, leaflets may be further defined and shaped, for example by connecting to the support layer and optionally to the stent, for example by sewing or stitching. Considering the size of a valve for use in a bodily conduit like blood vessels or arteries, the diameter of a tubular structure for making a leaflet assembly will be on the order of at most several centimeters. Such size may appear relatively small for (industrial) woven fabric production, but suitable weaving methods, weaving patterns and machinery are known in the art for such purpose; for example those generally referred to as narrow fabric weaving (systems) that are typically used for making tapes and ribbons, or for making implantable grafts. In such weaving equipment, typically movement of every warp thread can be individually controlled to make multiple layers, and various connections between layers. Further information on such weavings methods is available on the internet, for example on double weaving in the document available via http://www.cs.arizona.edu/patterns/weaving/webdocs/opr_rgdw.pdf.

The tubular woven fabric may be made using various fibers and yarns as warp and fill threads; including high-strength yarns such as UHMWPE multifilament yarn, resulting in thin and flexible yet very strong layers in the woven fabric. Forming the valve may further comprise attaching the leaflet assembly to a stent, for example with stitches. Whether commissures are formed in the weaving process or during later stitching, in both cases strong and durable commissures result at least at the connecting points between leaflets and supporting element and optionally stent at the outflow side of the valve, which are typically the places where most stress concentrates during valve opening and closure.

The invention also relates to a method of making a leaflet assembly as described in the method of making the valve, and to a leaflet assembly and a prosthetic valve obtainable by said methods, more specifically such prosthetic valve that can take a first form wherein the valve is open and a second form wherein the valve is closed, the valve comprising a leaflet assembly having at least two leaflets (3) attached to a supporting element (2), the leaflets having a free margin (5) that can move between a first position wherein the valve takes the first form and a second position wherein the valve takes the second form, wherein:
- the leaflet assembly is made from a single piece of seamless tubular woven fabric, made from warp and fill threads and having at least one stabilized edge, and
- the leaflet assembly has an inner layer forming the leaflets with the stabilized edge forming the free margin, and an outer layer forming the supporting element.

### Definitions

A *prosthetic valve* is a constitution of at least one leaflet and supporting element, wherein the leaflet is attached to the supporting element such that the leaflet can flex or hinge to provide an open as well as a closed position for the valve, and may optionally comprise a rigid or semi-rigid support, also called frame or stent.

A *leaflet assembly* is the combination of at least one leaflet and corresponding supporting element in a generally tubular configuration, and may be made from multiple pieces of material connected together or from one single textile structure (like a woven fabric). The leaflet is the movable part and is attached to the supporting element, also called graft or skirt, and together they define pockets that can be filled with fluid to close the valve.

A *commissure* is generally a point or line along which two things are joined; in anatomy of natural heart valves a commissure is the distinct area of junction between two adjacent valve leaflets and their supporting vessel wall. Within the present application the commissure refers to the attachment line or region from the outflow side between a leaflet and supporting element in case of a stent-less valve, and between leaflet and stent, and optionally supporting element for a stented valve. In addition to connections forming a commissure, there can be further connections between leaflet, supporting element and/or stent, for example further defining leaflet shape.

A *margin* of a leaflet is an edge.

*Coaptation* means abutting, contacting or meeting of a leaflet and a closure surface, such as another leaflet, to close the valve; *coaptation height* refers to the height or length of coaptation measured from the free margin in longitudinal direction of the valve, i.e. towards the bottom of the leaflet.

The *centre line* of a leaflet is a hypothetical line from the free margin at the centre of the valve to the nadir at the bottom of the leaflet, that is the lowest point defining the leaflet by connections to the supporting element. In case of a non-symmetrical valve with for example three leaflets, it is the line from the contacting or coaptation point of the three free margins to the nadir.

The *curvature height* characterizes the curvature in the leaflet of a valve as the largest orthogonal distance between the centre line and a straight line connecting the free margin at the centre of the valve and the nadir.

The *radius of curvature* of a leaflet is the radius of a circle that best fits a normal section of the curved surface of the leaflet in closed valve position.

An *elastic* material is a material that is capable of returning to its original shape after being deformed.

To *impose* a geometry on an object means that the geometry of this object is established by the creation of the object, as opposed to a geometry that can arise due to external forces applied to the object after creation.

*Inflow side* or bottom side of the valve means the side where fluid enters the valve when it is in open position, the opposite side is referred to as *outflow side* or top of the valve.

For something to *run parallel* with another thing means that both things predominantly extend in the same direction.

The *elongation at break* of a specimen is the elongation of that specimen recorded at the moment of rupture thereof under an applied load, expressed as a percentage of its original length. For sheet material, the elongation at break is often also called elongation at rupture or elongation at fracture.

A *yarn* is an elongated body having a length much greater than the width of its cross-section, typically comprising a plurality of continuous and/or discontinuous filaments, said filaments being preferably aligned substantially parallel to each other.

*Adjacent* means adjoining or nearest in position.

A *selvedge* (or selvage) is an edge of a woven structure wherein the threads that run in a direction perpendicular to the edge of the structure are not extending from the structure as free ends, but are continuous at the edge by returning into the structure. Selvedges are typically formed in fill (also called weft) threads during a shuttle weaving process, but may also be made with other techniques or in warp threads.

### Brief description of drawings

Figure 1 schematically shows various steps for forming a prosthetic valve using a method according to the invention.
Figure 2 schematically shows an alternate tubular woven fabric suitable for making a leaflet assembly and valve. Figure 3 schematically shows a way of making a selvedge perpendicular to warp direction of a fabric.
Figure 4 schematically shows various steps in another embodiment.
Figure 5 schematically shows an alternate tubular fabric.
Figure 6 shows a further embodiment of making a valve.
Figure 7 depicts another embodiment of leaflet assembly and valve.

All figures herein are schematic drawings only and not necessary to scale, and may not show all features or components for clarity reasons. Like reference numbers in different figures refer to like features.

### Detailed description

The method of the invention comprises a step of providing a single piece of fabric, more specifically a seamless tubular woven fabric having two open ends with at least one stabilized edge. A seamless tubular fabric is understood to have at least one circumferentially woven layer, without end-to-end connections of different pieces of fabric, like a seam or other connection. The advantage hereof is that thickness and properties of the layer are uniform, without weaker spots or other disruptions that could affect performance after implantation of a valve made therewith in a body. Such tubular fabrics can be woven with known techniques; like double weaving techniques wherein two or more interconnected virtually flat layers are made by interlacing multiple warp and fill (also called weft) threads, or techniques using special endless or (multi)ring-shaped warp beams.

In methods described in prior art often multiple woven textile structures, or pieces of woven textile structure, are used for forming a leaflet assembly comprising leaflets and supporting elements. Such methods may comprise forming each leaflet and supporting element from separate pieces of woven textile structure and then assembling and connecting the various pieces together, e.g. by sewing or stitching to make seams, before or during attaching them to a stent. In the present method multiple leaflets and supporting elements are made from a single piece of woven fabric, which also reduces the number of process steps in making the prosthetic valve.

The prosthetic valve made by the present method may be stent-less or may contain a stent attached to the leaflet assembly. A stent-less valve or leaflet assembly may be also used as a valved graft or grafted valve; meaning that the supporting element layer thereof can be attached to the wall of a blood vessel or artery and function as a graft to (partly) replace or reinforce a weak or aneurismal vessel. In such embodiment the outside of the leaflet assembly, the supporting elements layer, may be further treated to reduce permeability, e.g. by providing a coating or a further layer of material. A prosthetic valve with a stent provides some other advantages, for example the possibility of being implanted via minimal invasive techniques using catheter systems. In an embodiment the method thus further comprises attaching the leaflet assembly to a stent.

A tubular woven fabric can be made as a piece of fabric of a distinct length in a dis-continuous or piece-by-piece process, for example on a loom with warp threads attached to a beam from which it is detached once the desired length of fabric has been reached. A single piece of fabric can also be made in a continuous weaving operation by continuously feeding warp threads; resulting in a continuous tubular fabric, which is then cut into pieces of desired length. In both cases the obtained piece of fabric may have free ends of warp threads at the edges or extending from it, which edges can be stabilized to prevent unraveling or fraying. In the method of the invention this stabilization is at least done for the edge that later will form the free margin of a leaflet, and preferably all resulting cut edges of the piece of tubular fabric are stabilized.

In an embodiment the stabilized edge of the tubular fabric that will form the free margin is made by applying an after treatment, also called finishing step, for example including one or more steps like trimming, making a seam, stitching, gluing, or melt fusing threads at the cut edge. A preferred way of making a stabilized edge is hot cutting of woven fabric, especially of fabric made from warp and fill threads being thermoplastic polymer fibers. Such hot cutting can for example be done with a laser or with an electronic thermal cutter, also called hot knife, which moreover allows simultaneously cutting pieces from a fabric and stabilizing the edge by fusing thermoplastic fibers of such fabric in a controlled step.

In another embodiment a stabilized edge of the tubular fabric is woven as a selvedge. A selvedge is a self-finished or self-stabilized edge of a woven textile structure. A selvedge effectively refrains the textile structure from unraveling or fraying at such edge, and is the result of the weaving process rather than of an additional process step. In most woven textile structures selvedges run parallel to the warp threads and are created by the fill thread(s) looping back into the set of warp threads after exiting. A selvedge can also be woven in warp threads, by not continuously feeding warp threads or connecting warp threads directly to a warp beam, but by connecting warp threads via additional cords and/or hooks; for example using the Navajo or warp selvedge system as known in the art. By having the selvedge to form the free-margin of the leaflet, this free margin is provided as an inherently mechanically stable edge.

The prosthetic valve that is made with the method of the invention comprises two or more leaflets. Generally valves found in mammals, especially in the blood system, contain one, two or three leaflets; heart valves typically have two or three leaflets. In one embodiment a prosthetic valve is made that has two leaflets, with the second leaflet acting as a closure surface for the first leaflet and vice versa. In another embodiment the valve comprises three leaflets, each leaflet acting as a closure surface for the other two leaflets. Making prosthetic valves having more leaflets is likewise possible, but is more complex.

In an embodiment, a seamless tubular woven fabric having at least one stabilized edge is made by a double weaving process to result in a so-called flattened tubular fabric, flat-woven tubular fabric or hollow elongate fabric; as it results from a continuous fill thread crossing over from one set of warp threads forming a first layer to another set of warp threads forming another layer at each side edge after every interlacing. It is noted that for making a single layer, one-channel tubular fabric an uneven total number of warp threads is used to omit weaving errors, typically referred to as 'error corrected tubular weaving' in the art. Analogous weaving methods can be used to make a multi-layer and multi-channel fabric; like a double-walled tube wherein an outer tube is connected longitudinally to an inner tube along two or three cross lines, defining two or three outer channels and one inner channel (tube).

In an embodiment a single piece of fabric that is made by weaving warp and fill threads into a one-layer seamless tubular woven fabric is provided, that is a single tube or one-channel fabric; and the step of forming such fabric into a tubular leaflet assembly having an inner layer and an outer layer comprises partly inverting the piece of tubular fabric to form a tube-in-a-tube, wherein inner and outer layers are connected at one end at a fold line. Subsequent steps may include further connecting inner and outer layers , for example by stitching, to define and shape multiple leaflets, optionally combined with attaching the leaflet assembly to a stent. This is further explained in accompanying illustrating Figures by making a three leaflet valve as example; but which may similarly apply to making other valves.

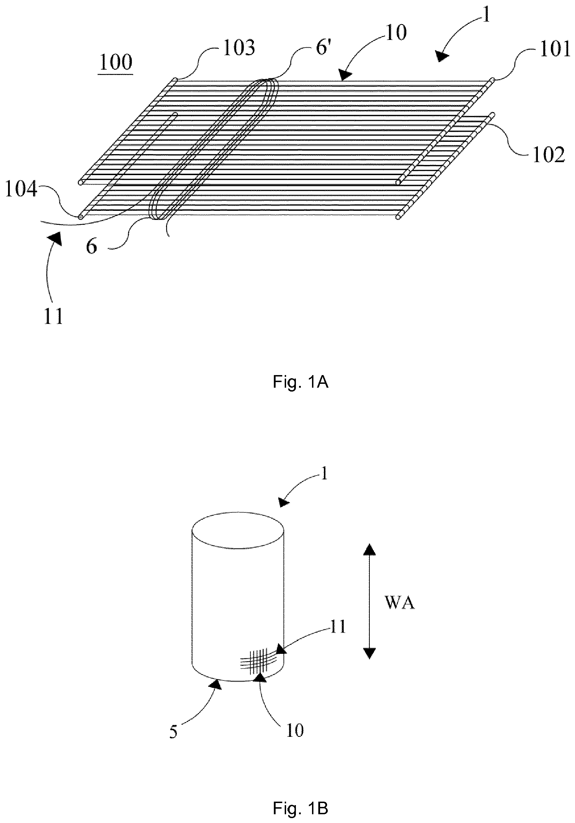

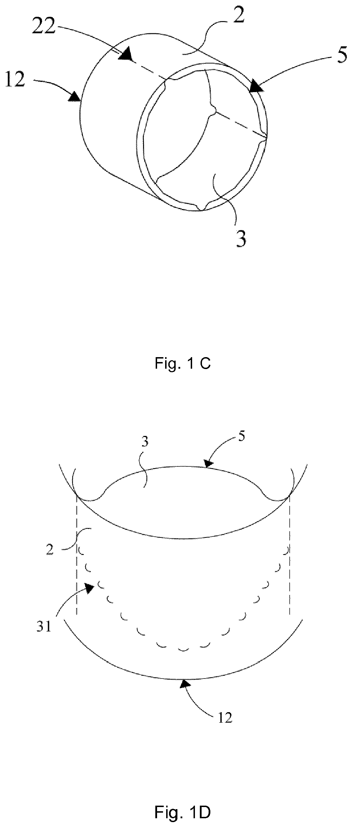

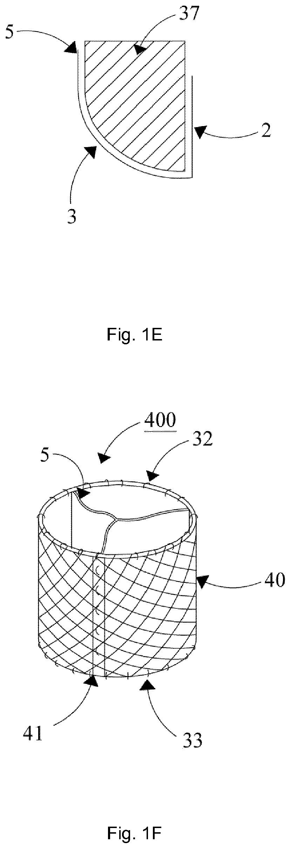

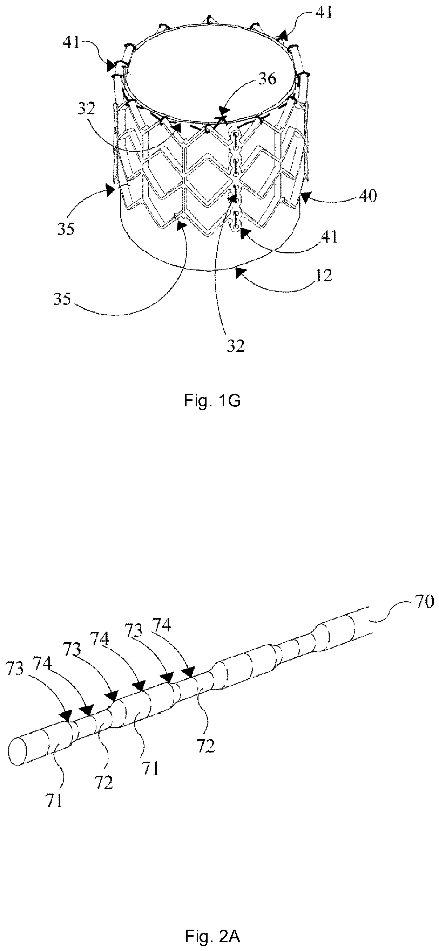

Reference is now made to Figure 1, comprising subfigures 1A- 1G, which schematically shows various steps of an embodiment of the method of forming a prosthetic valve starting from a substantially cylindrical seamless tubular woven fabric, wherein the open ends have substantially the same size or diameter. In Figure 1A a weaving loom 100 is depicted, the loom having four warp beams (or loom bars) 101, 102, 103 and 104. Warp threads 10 are provided between the upper two warp beams 101 and 103, and between the lower two beams 102 and 104. This way a textile structure having two stacked layers can be formed in one weaving process, using one loom set-up. For reasons of clarity, common other parts of the loom, such as the heald frames (or harnesses) with heddles to separate with a predetermined pattern warp threads in one layer (or in both layers) to form a clear space (or warp shed) through which (a shuttle or pick carrying) the fill (also called weft) thread can pass, and the optional bat (or reed) for pushing the fill thread against the fell of the cloth, are not shown. Warp threads may be attached to the beams (typical for a dis-continuous process), or may be continuously fed with beams 101 and 102 as guiding members, and 103 and 104 in such case representing a single fabric beam for receiving the fabric made. The fill thread 11 as shown in Figure 1A is woven in the upper layer of the textile structure 1 by interlacing the fill thread with each of the upper warp threads (e.g. forming a plain weave), and crosses at the edge of the upper layer to the lower layer, wherein it is woven until it reaches the other edge and then passes again to the upper layer via second crossing 6'. Note that for clarity the fold lines are made to look larger in the figure than in practice. The weaving process continues until the textile structure has the desired size. The result is a two layered woven textile structure comprising two layers connected along longitudinal crossings 6 and 6', by fill threads passing from the one layer to the other.

After the textile structure 1 is woven, it is released from the loom. Figure 1B shows the resulting tubular fabric in opened form, wherein warp threads run in the longitudinal direction (indicated by arrow WA). At least one of the edges is stabilized, e.g. by a thermal treatment; indicated by edge 5. Subsequently, the lower part of the tube as shown in Fig. 1B is inverted into the upper part, resulting in an inner layer 3 in the now outer layer 2, the layers 2 and 3 being connected at fold line 12. Then stitches 22 may be added to connect the layers 3 and 2 (in addition to fold line 12). By adding three lines of stitches 22 to this structure, layer 3 is divided in three separate sections corresponding to separate leaflets in the leaflet assembly, having stabilized edges 5 as free margin. Figure 1C shows the structure from a different viewpoint than in 1B, more clearly showing the inner layer 3 representing leaflets, and outer layer 2 forming supporting elements. The connection line 22 will form a commissure of the valve without stent; if a stent is subsequently attached line 22 may form part of the actual commissure.

In an optional step, as depicted in Figure 1D, additional stitches 31 are added, for example following a U-shaped curve, which further connect sections of layer 3 and corresponding sections in layer 2, to better define the leaflets or make a 3D-like shape (only one section shown). The connections made comprise, starting from the free margin, stitch 22 and stitch 31. Stitches 22 and 31 can also be continuous, i.e. stitches 22 may not extend over the full height of the valve, but may deflect and continue forming the U-shaped curve of stitches indicated as 31. This way, the leaflet and supporting element together form a pocket. By taking a position adjacent the supporting elements, the leaflets may open the ultimate valve, and by taking a position that extends away from the supporting elements, the leaflets may close the ultimate valve by contacting each other (coapting). These steps can likely be performed in the presence of a stent, thus connecting the leaflet by stitches to the stent.

Referring now to Figure 1E, in order to even better shape the leaflet and pocket a mold may be used. Before stitching connecting line 31, mold 37 may transpose the leaflet into shape, optionally by pulling the leaflet at edge 5 upwardly. This way, extra length is created between the nadir and the center of the valve along the leaflet. Another way of creating such extra length is to already weave layer 3 to be (locally) larger than layer 2, see hereafter. The steps as illustrated by Figure 1E can also be performed during or after connecting to a stent.

Figures 1F and 1G show an embodiment wherein the leaflet assembly is connected to a circular wire stent 40 to make valve 400. The leaflet assembly is placed within the stent and may be connected at its bottom to the stent with stitches 33, and at the top with stitching 32 connecting only supporting elements layer 2. This stitching 32 preferably continues to connect the leaflets and supporting elements with the three stent posts 41 (see also Figure 1G), such connection further forming the final commissure. The stabilized free margins 5 of the three leaflets are also depicted in Figure 1F. In this form, the valve 400 is closed by coaptation of the leaflets in neutral position. Would the free margins 5 be adjacent the supporting elements (i.e. adjacent the wall of stent 40), the valve 400 would be open. Some more details of the stent configuration and its posts 41 are depicted in Figure 1G. Knot 36 is made in suture 32, as connecting point for this suture after circumferentially connecting the fabric. In an alternative approach, stitches 33 are made at this stage; and temporary connections 35 may be used to keep the structure in place during suturing to posts 41 and can be removed thereafter. Figure 1G further shows an alternate embodiment wherein the leaflet assembly extends from the bottom of the stent, and this part may in a further step be folded to the outside of the stent and connected thereto, forming a cushioning layer on the stent. An advantage hereof may be smoother fitting to a vessel or artery upon implantation, e.g. using a catheter system.

In above exemplary embodiment a substantially cylindrical one-channel tubular woven fabric is used, at least one edge is stabilized, and subsequently the tube is partly inverted to make a tube within the tube. Inner and outer layers have substantially the same diameter, thus the free margins of the leaflets will in this case have substantially the same length as the corresponding supporting elements (e.g. equal to circumferential length 2πR, with R being the radius of the tube cross-section).

In an embodiment a tubular woven fabric for forming leaflets and supporting elements is provided, which fabric is made to have such size that after partly inverting and making connections between the layers a generally tubular leaflet assembly results wherein the free margins of the leaflets have at least the minimum length needed for closing the valve; i.e. for example the distance between the two ends of the free margin at the commissures via the centre of the valve in case of a substantially cylindrical leaflet assembly or valve having two or more leaflets. Preferably the free margin of a leaflet has excess length relative to said minimum length or distance. The circumferential length and diameter of the leaflet assembly and supporting elements at least correspond to the internal dimensions of the generally circular tubular stent of the valve during use (that is after possible expansion upon implantation). For example, in case of a substantially cylindrical valve with internal radius R, and having three leaflets of same size that are attached to the supporting element with even distribution between commissures the needed minimum free margin length would be 2R. By making leaflets having at least the same size as the supporting elements their free margin length would be at least 2πR/3; thus creating an oversize factor of at least about 1.05. Still more excess length can be obtained by forming oversized leaflets relative to actual size of the valve or its stent during use; which may be done during weaving the tubular fabric.

In general it was found to be advantageous to make a prosthetic valve wherein the leaflet free margins have a total oversize or excess length factor of at least 1.05, preferably at least 1.07, 1.09, 1.11, 1.13 or 1.15, and preferably of at most about 1.4, more preferably at most 1.3, relative to the minimum length needed for closing the valve (for example relative to the minimum length needed to bridge the distance between commissures via the center of the valve). Stated otherwise, the free margins preferably have an excess length of at least 5%, more preferably of at least 7, 10 or 15%, and of at most 40 or 30%. Such excess length of free margins is found to enable forming a relatively large closure surface between leaflets, i.e. in forming a significant coaptation height along the length of the free margins; and thus in effective closing of the valve upon reversed fluid flow and preventing significant regurgitation. A further advantage of the excess length is that it is not needed to make a leaflet assembly that precisely matches the diameter of a stent (after optional compression), but an oversized leaflet assembly can be used in a range of different stents (depending on desired minimum excess length of the free margin).

In an embodiment the prosthetic valve comprises leaflets that are made such that the leaflets, even without pulsatile load on the valve, can form a coaptation height of more than 0.1 mm along the length of the free margin. Preferably the coaptation height is at least 2, 3, 4 or 5 mm and at most 15, 13, 11, 10, 9, 8, or 7 mm, for example between 3 and 10 mm, preferably between 5 and 7 mm.

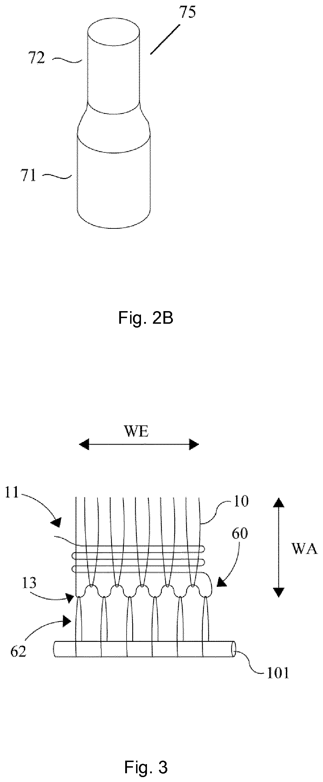

In a further embodiment a single piece of fabric is provided, which fabric is made by weaving warp and fill threads into a seamless tubular woven fabric having at least one stabilized edge, and having two open ends of different size or diameter; for example a conically shaped tube or a tube having a tapering or a tapered transfer zone between two substantially cylindrical parts of different diameter. Such tube having a tapered zone can for example be made by using a weaving process including gradual changes in the number of warp threads in the woven fabric, resulting in gradual changes in diameter-i.e. tapering- in the tube. Examples of suitable methods, which can be used to make continuous lengths of tubular woven with gradual changes in diameter, are for example described in US5800514 and US2014/0135906. A piece of tubular woven fabric having a first diameter at one end that is larger, preferably at least 2 or 5% larger, than a second diameter at the opposite end and with a gradual transfer of first to second diameter can be provided by cutting a suitable length from a continuous tubular woven fabric, and the ends are stabilized. Then the tube is partly inverted such that the part having the larger diameter will form the inner tube; meaning the free margins of the leaflets will have an excess length of more than 5%. Such steps are schematically represented in Figure 2. A part of a continuous tubular woven fabric 70 is shown in Fig. 2A, having multiple sections 71 of a first diameter and multiple sections 72 of a second diameter smaller than the first diameter, the sections 71 and 72 being connected via tapered zones 73. By cutting this tubular fabric 70 into pieces at lines 74 and stabilizing the cut edges, multiple pieces of seamless tubular woven fabric having one (or two) stabilized edge(s) result, one piece 75 being depicted in Fig. 2B. Analogously to the steps shown in Figure 1, such pieces can be formed into a leaflet assembly; be it that the leaflets therein will have more than 5% excess length depending on diameter difference between sections 71 and 72.

Figure 3 shows a schematical representation of a method of weaving a selvedge in warp threads, that is perpendicular to the warp direction WA of a fabric. Such weaving method may be used to make a single piece of tubular fabric having a selvedge as stabilized edge. In this case a stay is connected to the loom bar 101, the stay comprising multiple hooks 62. At the edge 5 the warp yarns 10 each form a loop, and each of these loops is fixed to the loom using the hooks of the stay. The fill thread 11 is interlaced with the warp threads in fill (or weft) direction WE. In this particular embodiment a cord 60 is used to fix the said loops to the hooks 62; which cord extends along the edge 13 through each loop of the warp threads. In this case, the cord 60 is a distal section of a warp thread and continues as fill thread 11, so no loose ends are adjacent edge 13. Using this method the warp threads at the edge form a loop, and thus are continuous; that is the edge 13 is formed as a selvedge. The selvedge in this case extends in the weft direction WE, perpendicular to the warp direction WA. This way of forming a selvedge is ideally suitable for forming endless or tubular textile structures, wherein a lateral edge will form the free margin of a leaflet in the ultimate valve. In another embodiment, the hooks connect the loom directly to loops of the warp yarns. To prevent a free end of the fill yarn, it is preferred to loop the fill yarn around one of the warp yarns and use the two ends of the yarn as individual fill yarns.

It was found that use of ultra-high molecular weight polyethylene (UHMWPE) yarns as fill threads was particularly advantageous when preparing a fabric with a selvedge parallel to the fill threads as these yarns tended to adjust transversely to fill the loops of the warp threads when stay or hooks were removed. It could be theorized (without wishing to be limited thereto) that this surprising finding for a yarn with very high strength and modulus is related to the combination of the low friction coefficient and bending flexibility of UHMWPE yarns.

In further embodiments, multi-layer and multi-channel seamless tubular woven fabrics, as illustrated in drawings hereafter, can be used for providing a single piece of fabric and for forming a leaflet assembly for use in the method of the invention.

In an embodiment a single piece of fabric that is made by weaving warp and fill threads into a flat-woven multi-layer tubular fabric comprising three or more channels is provided, and the step of forming such fabric into a tubular leaflet assembly having an inner layer and an outer layer comprises completely inverting, that is turning inside out, the piece of tubular fabric.

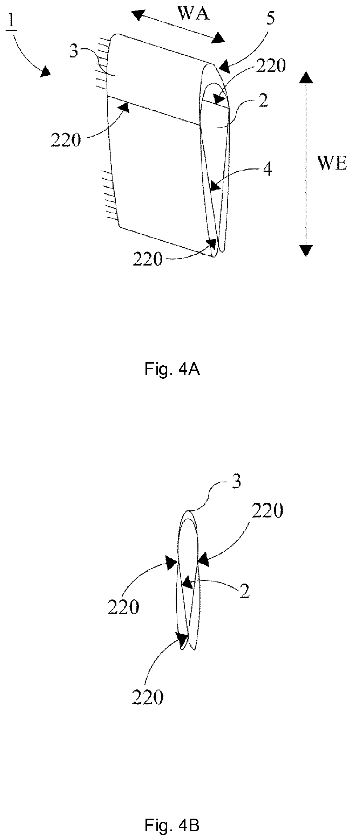

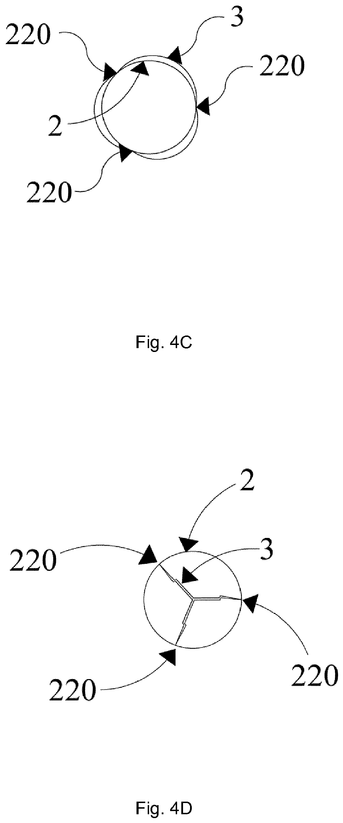

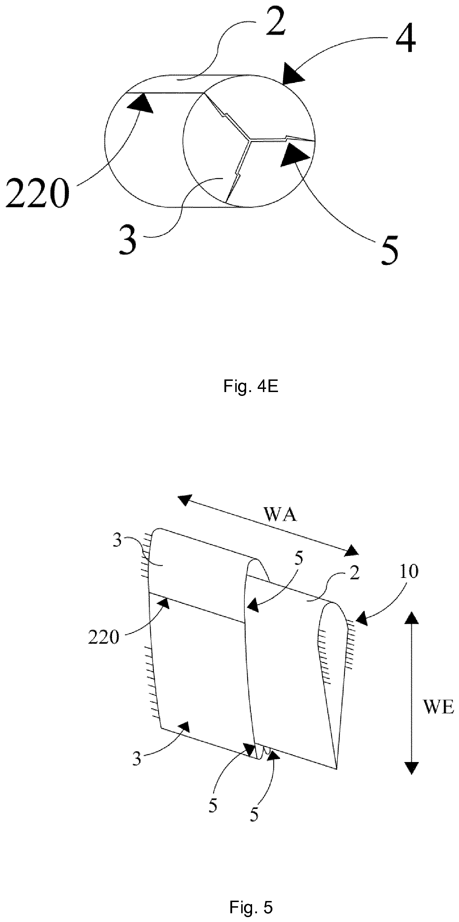

Referring to Figure 4, consisting of sub-figures 4A- 4E, various steps are schematically shown, wherein a seamless tubular woven fabric having 4 channels is used to make a prosthetic valve. Figure 4A (warp direction is indicated as "WA", fill direction as "WE") shows a single piece of woven fabric 1 consisting of an inner layer or tube, corresponding to the supporting elements 2 of leaflet assembly as depicted in Figure 4E, and an outer layer or tube corresponding to leaflet sections 3. The outer and inner tubes are connected along longitudinal cross lines 220, defining three channels and three sections between and in outer and inner layer; corresponding to three leaflets 3 connected to three supporting elements 2 after inverting. The cross lines are made during weaving by crossing of threads from one layer to another layer, such cross lines having similar strength as the fabric. In this embodiment the inner tube is made with selvedges 4, and the outer tube with selvedges 5; such that in the leaflet assembly the leaflets have selvedges 5 at their free margin. The outer tube in this fabric as woven has a larger circumferential length than the inner tube, meaning that the leaflets in the leaflet assembly after inverting the fabric will have excess length.

Figure 4B gives a front side view of the flattened woven fabric of Figure 4A. Figure 4C gives the same top or cross-sectional view, but then with the fabric being configured such that the inner layer 2 forms a cylindrical tube. The outer tube has three sections 3 that extend between the cross lines 220. In a next process step the textile structure of Figure 4C is completely inverted, that is turned inside-out, which leads to a structure as depicted in Figure 4D with leaflets in abutting or closed position. Stitches may be provided to further define leaflets and pockets, for example at the bottom side or following a U-shaped curve (not shown). Now the tubular woven fabric is a leaflet assembly with supporting elements 2 in the outer layer, and the inner layer forming the leaflets 3. An isometric view of this leaflet assembly is given by Figure 4E. Similar to the steps in Figure 1, the leaflets may be further defined and shaped, optionally combined with attaching to a stent. In other embodiments, an inner tube may have larger circumferential size than the outer tube, and inverting would not be needed.

In an alternative embodiment a single piece of fabric as depicted in figure 5 is used, wherein an inner tube 2 again defines a main part of the tubular fabric, but in contrast with the structure as shown in Figure 4A, this inner tubular layer extends over a longer distance than the outer tube. In this embodiment, the margin 5 of the outer tube later forming leaflets 3 is formed as a selvedge (for example using a circular loom bar and using a method as depicted in Figure 3). The edge of the inner tube can be woven as a regular edge and stabilized. This piece of fabric can be formed into a leaflet assembly analogously as described for Figure 4A. An advantage of the resulting assembly and valve is that the supporting element is longer, extending away from the actual leaflets. This extending part can be used for example to connect to the outside of the stent, e.g. as a cushioning layer. Alternatively, this leaflet assembly is not attached to a stent; but used without a stent as a valved graft, wherein the extending part of fabric can be used to attach the assembly to a vessel or artery wall.

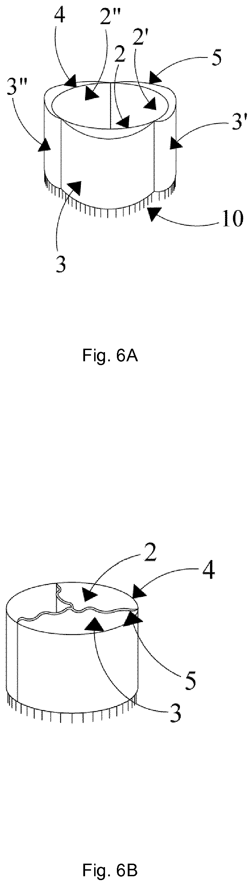

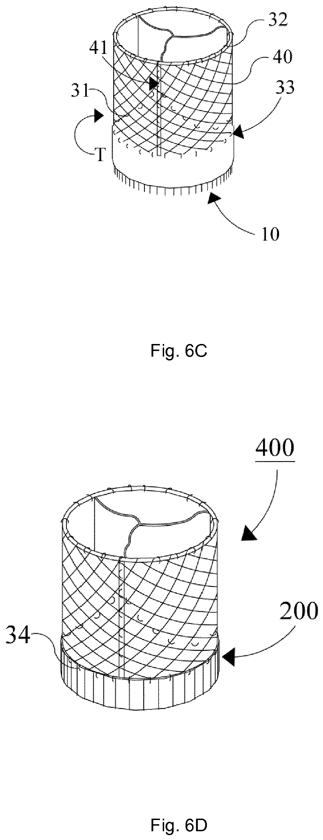

Figure 6, consisting of sub-figures 6A - 6D, schematically shows various steps in yet another embodiment of a method according to the invention. This method corresponds largely to the method described for Figures 4 and 5. At the bottom side of the piece of multi-layer tubular woven fabric, warp threads 10 are discontinuous after releasing the textile structure from the loom, as depicted in Figures 6A (the structure as woven and released) and 6B (the inverted structure or leaflet assembly in closed form). The edges 4 and 5 of the layers at the top are woven as selvedges (or otherwise made into a stabilized edge). In this embodiment a stent 40 having posts 41 is used to attach the inverted structure of Figure 6B to, as shown in Figure 6C. The stent used has smaller height than the leaflet assembly. Stitches 31, 32 and 33 are added, corresponding to the stitches as shown in Figure 1. Lastly, the part of the leaflet assembly extending at the bottom is turned around the stent as indicated in Figure 6C with the arrow T. As can be seen in Figure 6D, this way a rim 200 is formed by connecting the extending fabric to the stent with stitching 34. The rim can be used to suture the valve to the artery or aorta opening, and/or can provide a cushioning function.

In an alternative embodiment, a tubular woven fabric is made by using an endless warp beam, like a circular or triangular beam. Further, in addition to a single tube or one-channel woven fabric, also multi-channel or multi-layer tubular woven fabrics can be made by using multiple sets of warp threads and beams, specific designs of endless beams (that is beams having ends joined, like a circular loop), and/or specific crossing patterns of threads between the layers or tubular fabric layers.

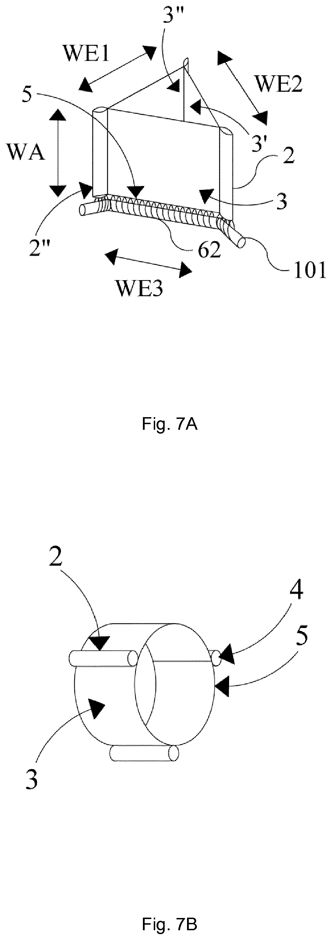

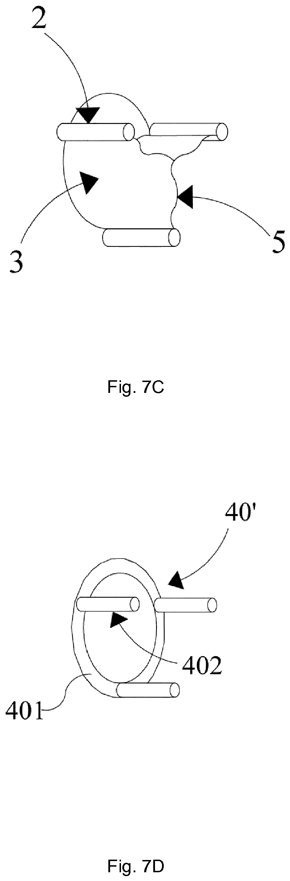

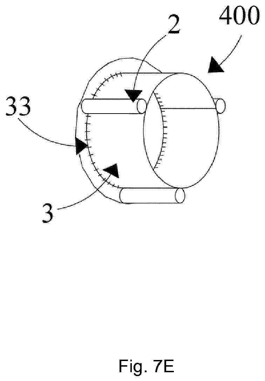

Figure 7, consisting of sub-figures 7A - 7E schematically shows another type of prosthetic valve that can be made using still another embodiment. In this method a piece of fabric is provided that is made by weaving a seamless tubular fabric having four parallel channels or tubes, wherein three sub-tubes, later forming supporting elements 2, are formed on the outer surface of an inner layer or tube having three sections, which will form leaflets 3. Leaflets 3 of this leaflet assembly are woven with a selvedge 5 at their free margin and supporting elements 2 are woven with a selvedge 4, using at one end the same method as depicted in Figure 3 (viz. using a stay with hooks 62 to fix the warp thread loops to the triangular loom bar 101). Alternatively, the edges are stabilized after weaving. For weaving this textile structure, three fill threads interlacing steps are needed, the different weft directions (WE1, WE2 and WE3) being indicated in Figure 7A. The sub-tubes can be used for attaching to a stent; for example by receiving three supporting pillars of a ring-shaped stent, and fixing the assembly to these pillars, to form valve 400. This leaflet assembly is shown in Figure 7B in its open form, and in figure 7C in its closed form.

Figure 7D shows stent 40', having a circular support ring 401 and three pillars 402, corresponding in shape to the three supporting elements 2 of the leaflet assembly as shown in Figure 7B. Valve 400 results from connecting the leaflet assembly to the stent 40' by sliding the supporting elements 2 over the pillars until the fabric abuts the circular support 401; and then connecting to the circular support with stitching 33 to form prosthetic valve 400 as shown in Figure 7E. The circular support makes sure that the valve 400 maintains its shape.

In a further embodiment (not shown) the sub-tubes or supporting elements 2 have a narrowing (or optionally even a closure) on their ends adjacent the free margin 5. This way, the textile structure is easier to position over the pillars, since the narrowing prevents the valve collapses in longitudinal direction. Alternatively other connecting means like stitching may be used to fix the supporting elements to the pillars.

The piece of fabric that is used in the method of the invention is made by weaving warp and fill threads into a seamless tubular woven fabric as discussed above. The weaving pattern applied during weaving the one or more layers of the fabric is not found to be particularly critical, and the skilled person will be able to select a pattern in combination with selected threads to obtain desired properties with some experiments. Typically, woven fabrics with commonly used patterns like plain, twill or basket weave patterns are found to provide good performance. For making fabrics having 3D-like shape, especially for making shaped leaflets, also a combination of different weaving patterns may be applied. By using locally a different weaving pattern, for example resulting in a more dense fabric structure, different shapes may result to form e.g. a curved surface as part of the weaving process.

In the method of the invention a tubular woven fabric is used, which fabric comprises layers of such thickness and is woven with such warp and fill threads that a strong yet flexible and pliable fabric results, to enable high responsiveness of leaflets moving from open to closed positions in response of pressure differences over the valve, and effective closing by the leaflet abutting with a closure surface and forming sufficient coaptation. In an embodiment the fabric contains one or more layers with single layer thickness of about 20-200 µm. Preferably layer thickness is at most 180, 150, 140, 130, 120, 110 or 100 µm and at least 30, 40, 50 or 60 µm for good performance. In embodiments the tubular woven fabric contains layers with thickness between 40 to 150 µm, or having a thickness of between 50 to 100 µm.

In the method of the invention various types of fibers can be used as warp and fill threads, including natural or biological, as well as synthetic fibers. Threads may be formed from monofilament or multifilament yarn. More than one type of fiber may be used as warp and fill threads, and warp and fill threads may differ from each other. For making fabrics with uniform properties and less complicated production use of one type of fiber for warp or fill, or for warp and fill threads may be preferred. In an embodiment both warp and fill threads comprise at least 80 or 90 mass% of one type of fiber, and preferably consist essentially of one type of fiber. Suitable synthetic fibers include yarns made from thermoplastic polymers; for example from polyesters like PET, from polyurethanes, or from polyolefins like PE or PP. In an embodiment the textile structure comprises yarns having an elongation at break of at most 10%. In a further embodiment the threads have a linear density of less than 120 dtex, preferably a linear density of less than 100, 80, 60, 50, 40, 30, 20 or even 15 dtex, preferably linear density of at least 5, 7, or 10 dtex; for example a linear density of between 5 and 30 dtex, or between 7 and 15 dtex. Applicant found that there are advantages in applying fabrics made from thin yarns for making a prosthetic valve regarding flexibility and responsiveness of the leaflets (note: although dtex is not a parameter that denotes actual dimension or spatial length, in practice it corresponds to yarn diameter since most synthetic and natural materials for making yarns have a density of about 1 kg/dm³).

In another embodiment the warp and fill threads in the woven fabric comprise or are made from high-performance polymeric yarn, especially multi-filament yarn having high tensile strength or tenacity of at least 1 GPa. Examples include carbon, aromatic polyamide, aromatic polyester, and ultra-high molecular weight polyolefin yarns.

In a further embodiment the warp and fill threads comprise ultra-high molecular weight polyethylene (UHMWPE) fibers, preferably the threads comprise at least 80 mass% of UHMWPE yarn, more preferably the warp and the fill threads substantially consist of UHMWPE multifilament yarn. Such yarns have been found to be ideally suitable for use in woven fabric for making leaflets and supporting elements of a valve prosthesis. The UHMWPE yarns are durable, can be made with the desired mechanical properties and a medical grade is commercially available, which medical grade is hardly immunogenic. In particular, it is preferred to use UHMWPE yarn that has an intrinsic viscosity (IV) of at least 5 dl/g, preferably at least 10 dl/g, more preferably at least 15 dl/g. Preferably, the IV is at most 40 dl/g, more preferably at most 30 dl/g, even more preferably at most 25 or 20 dl/g. IV is determined according to method PTC-179 (Hercules Inc. Rev. Apr. 29, 1982) at 135°C in decalin, the dissolution time being 16 hours, with DBPC as anti-oxidant in an amount of 2 g/l solution, by extrapolating the viscosity as measured at different concentrations to zero concentration. Particularly preferred are gel-spun UHMWPE yarns, which typically have a Young's modulus of at least 30 or 50 GPa and a tenacity of at least 1 or 2 GPa. Tensile properties of UHMWPE yarn are defined and determined at room temperature, i.e., about 20°C., on multifilament yarn as specified in ASTM D885M, using a nominal gauge length of the fibre of 500 mm, a crosshead speed of 50%/min and Instron 2714 clamps, of type "Fibre Grip D5618C". On the basis of the measured stress-strain curve the modulus is determined as the gradient between 0.3 and 1% strain. For calculation of the modulus and strength, the tensile forces measured are divided by the titre, as determined by weighing 10 metres of yarns; values in GPa are calculated assuming a density of 0.97 g/cm³. Preferably the yarn used comprises at least 80 or 90 mass% of UHMWPE filaments, or consists essentially of UHMWPE filaments. A preferred example of an UHMWPE yarn is Dyneema Purity® yarn obtainable from DSM, The Netherlands. This type of UHMWPE yarn is a medical grade yarn available in low dtex versions, the yarns typically having an elongation at break of about 2 to 4%. The ultra-high molecular weight polyethylene may be linear or branched, although preferably linear polyethylene is used due to the very high tenacity and modulus obtainable by stretching during manufacturing of the yarn. Linear polyethylene is herein understood to mean polyethylene with less than 1 side chain per 100 carbon atoms, and preferably with less than 1 side chain per 300 carbon atoms; a side chain or branch generally containing at least 10 carbon atoms. The number of side chains in a UHMWPE sample is determined by FTIR on a 2 mm thick compression moulded film, by quantifying the absorption at 1375 cm using a calibration curve based on NMR measurements (as in e.g. EP0269151).

Woven fabric made from such UHMWPE yarn provides good biocompatibilty to the prosthetic valve, and is very flexible, thus enabling fast response of the leaflet under pulsatile load. The flexible leaflets can also easily align with the supporting elements, thus creating an orifice approaching the dimensions of stent and supporting elements; also inducing less load on the commissure. Furthermore, it was found that the use of such thin yarns tends to lead to woven textile structures having relatively low pore size, and favourable blood compatibility. Durability of the valve may be further improved, for example by making stronger connections or attachments by stitching through multiple layers of fabric in forming a commissure, which is possible as the thin fabrics are flexible enough to allow folding of layers.

It is noted that use of such woven fabric made from UHMWPE multifilament yarn is against the teaching of prior art to use a material that allows elastic stretching of about 15%, to mimic the stretch behavior of natural leaflet material. As UHMWPE yarns typically have a low elongation at break and high resistance to stretching (high modulus), a woven fabric made therefrom will also be a relatively low-stretch material. It is believed to be a further advantage of the present method that use of such a textile structure may provide more durable leaflets and valve after implantation, not only from a mechanical point of view but also since stretching an object may induce collagen growing over this object. The low stretch characteristics of present leaflets thus reduce or even minimize the impetus of potential collagen or connected tissue overgrowth, that would otherwise result in leaflet thickening and loss of mobility and possibly induce focal thrombi or other vegetation. In general, tissue overgrowth or fibrosis may lead to leaflet compaction, which will result in valvular incompetence.

In the method according to the invention, stitches can be used to make the leaflet assembly as such and to attach the leaflet assembly to a stent, a.o. to form the commissures. Such stitches are preferably made using a yarn or suture material that has similar strength properties as the yarn of the woven fabric. In preferred embodiments, stitches are made using a yarn or a suture of suitable size or linear density, which comprises at least 80 or 90 mass% or consists essentially of UHMWPE yarn as defined above to ensure strong and durable connections and commissures.

In another embodiment forming the leaflet assembly may further comprise a step of shaping a leaflet by contacting with a mould of desired shape, optionally heating the mould to a temperature of 3-60 °C (preferably 5-40 °C) below the melting point of the thermoplastic polymer, e.g. UHMWPE (see ISO11357-3 for a determination of the melting point of a polymer), optionally creep forming the textile structure (i.e. altering its dimensions), and submitting it to a controlled relaxation and/or plastic stretching to conform to at least a part of the mould. Such thermal forming process is for example described in WO2010/020660. With this embodiment a geometry is imposed to the leaflet, for example to create certain curvature or to meet certain clinical demands.

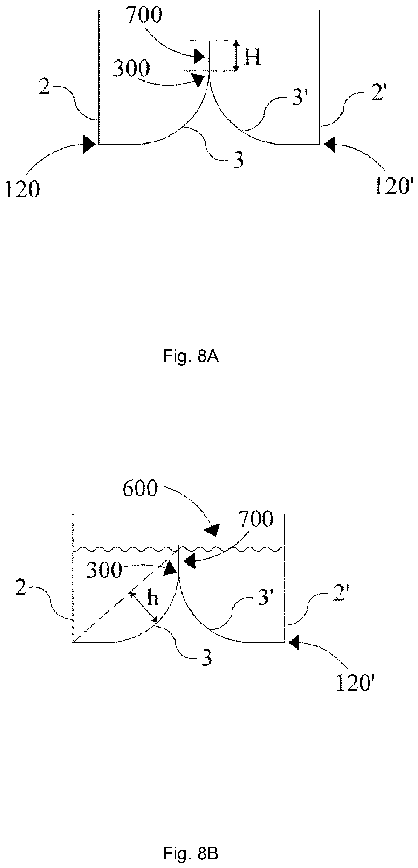

In Figure 8A a cross section of a leaflet assembly for a prosthetic valve having two opposing leaflets is shown. The leaflets 3 and 3' have a geometry in neutral position without pulsatile load that enables them to abut each other along the length of the free margin, and therewith form a coaptation 700 with a coaptation height H at this cross section. The coaptation height H extends with a minimum of 0.1 mm (the bottom of which is indicated with reference number 300) over the length of the free margin of each of the leaflets, possibly becoming even larger towards the commissures depending on commissure length. The geometry also comprises per leaflet a convex surface that extends between the top of the closure surface and the respective connections to supporting elements, of which nadirs 120 and 120' are indicated. Each convex surface bulges away from the respective supporting elements 2 and 2'. In Figure 8B it is shown that by a hydrostatic pressure, for example created by filling the pockets with water 600 as indicated, the imposed geometry and the coaptation height including formation of a closure "ribbon" having the length of the free margins can be inspected more easily and its dimensions estimated. It is noted that due to excess length of the free margin (more textile length then actually needed to span the distance between supporting elements and to coapt), it might be that at some spots when closing the valve by filling it with water, there is a wrinkle or small opening (a channel) in the closure surface. Such opening however is not persistent and will be closed in actual use by pulsatile load. Height h is the largest orthogonal distance between the line connecting free margin and nadir, and the curved surface of the leaflet. In another embodiment the leaflet comprises a convex surface, wherein the height h at the centre line of the leaflet is more than 1 mm, preferably more than 2, 3 or 4 mm most preferably about 5 mm. A maximum value is inherently dependent on the outer dimensions of the valve itself, but is typically about 10-15 mm, for example 10, 11, 12, 13, 14, or 15 mm. It is believed that an imposed convex geometry with this particular shape leads to less stress in the leaflet material and possibly less tension on the commissures.

In yet another embodiment the method further comprises steps of decreasing the permeability of at last part of the woven fabric by applying a coating or optionally arranging the structure in a mould, heating to a temperature of 3-15 °C below the melting point of the thermplastic polymer of warp and fill threads, preferably UHMWPE, and holding at a temperature of 3-15 °C below the melting point for 10 seconds to 2 hours to impart a partial connection between adjacent filaments and/or yarns in the fabric. Depending a.o. on the cross section of the yarns and their arrangement in the textile structure (for example type of weave), it can be advantageous to decrease the permeability of the textile structure.

The method of making a prosthetic valve may further comprise forming the valve by attaching the leaflet assembly to a stent. Such stent or frame is a rigid or semi-rigid structure typically comprising a rigid member, and often is of ring or cylindrical shape. Suitable materials for making a stent include rigid polymers, fiber-reinforced polymers, metals and their alloys, ceramics and combinations thereof. Suitable rigid polymers include polyacetals, dextroplast, polyurethane, polyethylene, polysulfones, polyethersulfones, polyarylsulfones, polyetheretherketones, and polyetherimides. Suitable metals include biocompatible metals, such as, stainless steel, titanium, cobalt alloys, such as Elgiloy®, a cobalt-chromium-nickel alloy, and MP35N, a nickel-cobalt- chromium-molybdenum alloy, and Nitinol®, a nickel-titanium alloy. In addition, stents can be produced from ceramic materials, such as pyrolytic carbon, silicon carbides or metal carbides, hydroxyapatite and alumina. Suitable stents can also be produced from carbons such as graphite. Preferably, a stent is at least partly made from a super elastic alloy, or a shape memory alloy, such as Nitinol®, that is available as a super elastic material, as well as a shape memory alloy. Such a stent allows to easily insert the valve prosthesis into the body in a desired position, for example using a catheter system. Before insertion, the self-expandable stent is brought to a first (relatively low) temperature at which it has a compact configuration. This compact configuration allows to easily insert the stent (and the valve in conjunction therewith) into the body, using minimal invasive surgery. After positioning the stent, the shape memory alloy will heat up to the body temperature and change phase, thereby changing its shape into a larger diameter. For Nitinol® for instance, a phase change will occur between an austenitic phase and a martensitic phase. As a result the stent will expand and thereby create a clamping force against surrounding tissue. In another configuration, Nitinol® is super elastic and can be elastically deformed up to material strains of about 10%, thus deformation of a valve towards a compact shape is possible, still allowing elastic deployment to the final shape after placement.

The invention also relates to making a leaflet assembly as described in the above methods and figures, and to a leaflet assembly and to a prosthetic valve obtainable with or obtained by the above described methods, more specifically such prosthetic valve as defined in the embodiments listed below and by the claims.

The invention will now be further illustrated using the following nonlimiting experiments.

### Experiment 1

This experiment describes making a prosthetic valve and experiments wherein such valve is tested in vitro and used as a pulmonary valve prosthesis by implanting in sheep. In this example, each valve is made with the method described below, which is basically makes a tubular structure from a single piece of woven fabric by connecting ends via a seam. Although this is not a seamless tubular fabric, the skilled person will understand from these illustrative experiments that a prosthetic valve made according to present invention as elucidated by the description and drawings will at least show similar performace characteristics. It is noted that a number of below described steps are correspondingly shown in Figure 1.

A fabric was woven from Dyneema Purity® TG 10 dtex UHMWPE multifilament yarn (available from DSM, The Netherlands) as a 2 by 2 twill weave, with longitudinal selvedges and with a density of 458 warp yarns per inch and 223 fill yarns per inch, and with a layer thickness of 0.00314 inches (80 µm). The fabric was folded double in to a two-layer structure, with a length of 90 mm and a width of 21.5 mm. A cylindrical stent having the design as shown in Figure 1G, made of electromagnetically polished stainless steel 304 was used. It had an outer diameter of 25 mm, an inner diameter of 23 mm and a height of 17 mm. For making stitches, two kinds of suture thread was used: Maxbraid PE 3-0 suture blue with tapered needles (available as MPC 900252 from BIOMET MERCK LTD), here beneath referred to as Suture A, and Maxbraid PE 4-0 suture blue with tapered needles (available as MPC 900244 from the same supplier), here beneath referred to as Suture B. Both sutures comprise UHMWPE yarn.

The pulmonary valve was made as follows. In order to create a coaptation height of about 6 mm over the length of the free margins of the leaflets, extensive free margin length was created. The free margin length was oversized by following steps:
1. The leaflet free margin length in the textile structure as woven will be inherently equal to the supporting element length, as the two layers have the same length. The distance between the edge of the supporting element formed as a cylinder and the middle of the valve being its radius R, the total length needed for 3 leaflets bridging this distance is 6R, whereas the length of the supporting element is 2πR. This creates an inherent excess length factor for the leaflet of 2πR/6R= 1.05.
2. The two layer woven fabric is initially wrapped around (i.e. to the outside of) the 25 mm stent and the ends perpendicular to the free margin of the leaflets are sutured together. Subsequently the cylindrical textile structure is placed inside the stent of inner diameter 23 mm and fixed to the stent with UHMWPE sutures. This creates an excess length factor of 25/23= 1.09.
3. In this example the actual prosthetic heart valve size is 23 mm for implantation, therefore the stent of 25 mm outer diameter is radially compressed to 23 mm. This way the inside diameter of the stent where the supporting element and leaflet is fixed to is reduced from 23 mm to 21 mm. This creates an excess length factor of 23/21 = 1.10.
The total excess length factor of leaflet free margins created this way is πx25 / 3x21 = 1.25. The excess length thus created is about 25%.

As indicated here above, the two-layer woven fabric is tightly wrapped around the stent, initially being used as mold, and the four layers at the closure are sutured together with Suture A starting at the outflow side of the fabric/stent combination by creating a knot, leaving about 2 cm loose end and a long end which is used to create a stitch line towards the inlet side of the fabric/valve combination. The stent/mold is removed carefully, and the tubular textile structure is placed inside the stent. The orientation of the warps of the leaflets and supporting element are perpendicular to the longitudinal central axis of the stent and commissural stent posts, ergo the fill yarns are in parallel to the central axis and commissural stent posts. The Suture A is then guided across fringe and stent post holes from inlet side towards outlet side (correspondingly shown in Figure 1G), thus fixing the stent post to the supporting element and leaflet at a length of about 9 mm. At the top of the post (outflow side) suture A is used to fix the edge of the supporting element to the stent in a continuous way by taking locked bites at the bended ends of the stent (the commonly known "Method of Blalock" using a festooning suture line). The end of the suture A is tied to its beginning at the knot's loose end. The textile structure is temporarily fixed to the remaining commissural stent posts in a 120 degree fashion thus dividing it in three parts with about the same free margin length, to keep the structure in place during next steps; after which the temporary fixations can be removed.

A second suture B is used to complete attaching of the textile structure and create the actual leaflet assembly within the stent, by stitching to the two remaining stent posts with a length of about 9 mm, and by stitching leaflet layer to the supporting element layer and stent to create the valve cusps. Prior to suturing, the free margin of all three individual leaflets were pulled up 3 mm in the middle of the free margin at the expense of length of the supporting element at the inflow side thus creating an arch of woven fabric between commissural posts elevated over the plane of the stent outflow side. Together with the aforementioned excess length this results in about 6 mm coaptation height in the center of the heart valve, and is likely even higher towards the commissures of about 9 mm. A mold (a negative form taken from a human aortic valve) is used for further sizing and shaping the belly of the leaflet (also shown in Figure 1E). The leaflet assembly is temporarily sutured in the middle between the posts at the inflow side to maintain this configuration during next step. From this point suturing is started, as correspondingly shown in Fig 1G. At the top of the post the leaflet and supporting element are taken double with two encircling bites. The leaflet sheet is pulled a little bit backwards over the top of the stent and is fixed by the suture. The course of the suture line of the leaflets (U-shaped) is also guided by the shapes of the stent and mold. The end of the suture is tied to the loose end left at the knot of the beginning of suture B. The resulting leaflets had a convex surface at the centre line of these leaflets with a radius of curvature of about 12 mm without pulsatile load. This was estimated to represent a distance h as depicted in Figure 8B along the centre line with a height h of about 5 mm. The textile structure extends a few millimetre from the stent at the inflow site, as also shown in Fig. 6C, which for example can be used to attach the valve to vessel or artery wall upon implantation. The leaflet assembly is further connected with sutures to the lower part of the stent, and the temporary sutures are removed.

After this fixation of leaflet assembly, the stent of the valve is compressed from 25 mm diameter to 23 mm diameter and sterilized by using ethylenoxide sterilization.

Performance of valves made as described above was tested both in vitro and in vivo. Mechanical and functional testing of the prosthetic heart valve was performed in a simplified mock circulation. A BVS 5000 circulatory assist device (Abiomed, Danvers, MA, USA) was included in a closed loop circuit having a reservoir and a return conduit. The heart pump bladder was driven by an Intra Aortic Balloon Pump (Maquet, Rastatt, Deutschland) with a frequency of 80 beats/min and output of 3600 cc/min, while afterload at the outflow side of the heart pump was set to 80 mmHg using a water column. In an initial test the standard valve of the heart pump at the outflow side was replaced by a valve constructed with three single leaflets made from woven fabric of 55 dtex UHMWPE yarn mounted in a transparent plastic conduit to study its open and closure behavior. This pilot valve sustained over 4 weeks (3.571.200 cycles) while remaining competent without deterioration of the woven leaflets. Based on this experience, a valve constructed as above (based on leaflets from woven fabric of 10 dtex UHMWPE yarn), was tested under equivalent physiologic loading conditions of the systemic human circulation, cumulatively during over 120 days (13.824.000 cycles). The valve opened fully into an optimal effective orifice, having commonly known vertical position of vibrating leaflets in parallel to the fluid stream, and closed while visually not revealing closure defects along the coaptation line of meeting free margins of leaflets, except from a tiny central hole of about 0,5 mm. Visual inspection after testing revealed a completely intact valve geometry; leaflets showing no fraying at the free margin or any other disruption or defects. All the suture lines as described above, as well as the knots were intact.

The prosthetic pulmonary valves were also implanted in adult sheep models (bread "swifter", body mass 55-70 kg) on the beating heart, while using an extra-corporeal circulation machine. Access to the pulmonary artery was achieved through left thoracotomy 3rd-4th i.c.s. The pulmonary artery was incised longitudinally, whereafter the native leaflets were cut out. Three positioning stitches of 5-0 Prolene® were used to pull on the commissural native posts. The valve was sutured into the pulmonary artery on the supra annular level (plane top of native commissures) using 5-0 Prolene®. The pulmonary artery was closed in linear fashion.

Echocardiography showed normal leaflet function without valvular or paravalvular regurgitation, apart from some occasional minimal regurgitation in the centre of the valve. The wound was closed and the sheep was taken to stables for recovery.

All treated sheep remained stable, without any adverse clinical signs up to 6 months observation periods. After this period the leaflet function was assessed again. Echocardiography showed adequate leaflet function with minor to moderate valvular but no paravalvular regurgitation, and there was no change in effective orifice since the day of implant. After this, the valves were taken out of the sheep for inspection. The leaflets and supporting elements were overgrown with tissue, but this appeared to be a very thin layer of fibroblasts and endothelial cells without histological and radiological signs of tissue calcification, and with a maximum thickness (including the leaflet) of 250 µm at the free edge with increasing amount of streamlining repair tissue towards the nadir. The mechanics of the valve appeared to be unaltered, all sutures were in place without fractures and the free margin of the leaflets appeared to be completely intact as originally made. No signs of fraying or other anomalies could be detected. The inventors are not aware of other studies using a prosthetic valve having leaflets made from a fabric woven from synthetic fibers, and wherein animals having such implanted valve survived a 6 months period without complications.

### Experiment 2

A prosthetic aortic valve to be implanted in the systemic circulation was made analogously to Experiment 1 with some modifications. The supporting element was prepared by taking out three half-moon pieces of fabric (facing the sinus valsalva in the human or animal aorta) to allow blood supply to flow into the coronary ostia. The remaining edge of the supporting element was fixed to the leaftlet according to corresponding suture line of the U-shaped cusp suture line (facing the sinus valsalva). A second suture was used to complete attaching of the textile structure and create the actual leaflet assembly within the stent, by stitching to the stent posts with a length of about 9 mm, and by stitching the leaflet layer to the supporting element layer and stent to create the valve cusps.

The valve was subsequently constructed in similar way as the pulmonary valve described here above. When completed, an additional sewing cuff of braided UHMWPE yarn was sutured with MaxBraid™ 3-0 UHMWPE (available from Teleflex, Limerick, Ireland), in an everted fashion using the Blalock stitch configuration.

Valves were implanted in adult sheep models (bread "swifter", body mass 65 kg) on the arrested heart under support of extra-corporeal circulation. Access to the aortic root was achieved through left thoracotomy 3rd-4th i.c.s. The pulmonary artery was dissected and pulled aside to allow transverse incision of the aorta. Classical implant was performed under cardiac arrest using a running suture Prolene® 5-0. The aorta was closed with a pericardial patch and the heart was defibrillated thereafter. The heart lung machine was disconnected. Echocardiography showed normal leaflet function without valvular or paravalvular regurgitation.

## Claims

1. A method of making a prosthetic valve (400) that can take a first form wherein the valve is open and a second form wherein the valve is closed, the valve comprising a leaflet assembly having at least two leaflets (3) attached to a supporting element (2), the leaflets having a free margin (5) that can move between a first position wherein the valve takes the first form and a second position wherein the valve takes the second form, the method comprising:
o providing a single piece of fabric, made by weaving warp and fill threads into a seamless tubular woven fabric having at least one stabilized edge, and
o forming the piece of fabric into a leaflet assembly having an inner layer forming the leaflets with the stabilized edge forming the free margin, and an outer layer forming the supporting element.

2. The method according to claim 1, further comprising attaching the leaflet assembly to a stent.

3. The method according to claim 1 or 2, wherein the single piece of fabric is made by cutting a continuous seamless tubular woven fabric into pieces of desired length, and by stabilizing at least one of the resulting cut edges.

4. The method according to any one of claims 1-3, wherein the warp and fill threads are thermoplastic polymer fibers and the stabilized edge forming the free margin is made by melt fusing.

5. The method according to any one of claims 1-4, wherein the single piece of fabric is made in a dis-continuous process and the stabilized edge forming the free margin is woven as a selvedge.

6. The method according to any one of claims 1-5, wherein the prosthetic valve has two or three leaflets.

7. The method according to any one of claims 1-6, wherein the free margin of a leaflet has excess length relative to the minimum length needed for closing the valve of at least 5%.

8. The method according to any one claims 1-7, wherein the single piece of fabric is made by weaving warp and fill threads into a one-channel seamless tubular woven fabric, and forming the fabric into a tubular leaflet assembly comprises partly inverting the piece of fabric to form a tube-in-a-tube.

9. The method according to any one of claims 1-7, wherein the single piece of fabric is made by weaving warp and fill threads into a multi-layer tubular fabric comprising three or more channels, and forming such fabric into a tubular leaflet assembly comprises inverting the piece of tubular fabric.

10. The method according to any one of claims 1-9, wherein the fabric is made with plain, twill or basket weave pattern, or by a combination of different weave patterns.

11. The method according to any one of claims 1-10, wherein the fabric contains one or more layers with single layer thickness of 20-200 µm.

12. The method according to any one of claims 1-11, wherein warp and fill threads comprise at least 80 mass% of one type of monofilament or multifilament yarn.

13. The method according to any one of claims 1-12, wherein the warp and fill threads have a linear density of less than 120 dtex.

14. The method according to any one of claims 1-13, wherein the warp and fill threads comprise yarn of thermoplastic polymer.

15. The method according to claim 14, wherein the warp and fill threads comprise ultra-high molecular weight polyethylene (UHMWPE) yarn.

16. A leaflet assembly for a prosthetic valve as obtainable by the method according to any one of claims 1-15, the leaflet assembly having at least two leaflets attached to a supporting element, the leaflets having a free margin that can move between a first position and a second position, wherein the leaflet assembly
o is made from a single piece of seamless tubular woven fabric made from warp and fill threads and having at least one stabilized edge, and
o has an inner layer forming the leaflets with the stabilized edge forming the free margin, and an outer layer forming the supporting element.

17. A prosthetic valve as obtainable by the method according to any one of claims 1-15, which valve can take a first form wherein the valve is open and a second form wherein the valve is closed, and comprises a leaflet assembly having at least two leaflets attached to a supporting element, the leaflets having a free margin that can move between a first position wherein the valve takes the first form and a second position wherein the valve takes the second form, and wherein the leaflet assembly
o is made from a single piece of seamless tubular woven fabric, made from warp and fill threads and having at least one stabilized edge, and
o has an inner layer forming the leaflets with the stabilized edge forming the free margin, and an outer layer forming the supporting element.

18. The prosthetic valve according to claim 17, further comprising a stent attached to the leaflet assembly.

19. The prosthetic valve according to claim 17 or 18, wherein the valve comprises two or three leaflets, each leaflet acting as a closure surface for the other leaflet(s).

## Patentansprüche

1. Verfahren zur Herstellung einer Prothesenklappe (400), die eine erste Form, in der die Klappe geöffnet ist, und eine zweite Form, in der die Klappe geschlossen ist, annehmen kann, wobei die Klappe eine Segelanordnung umfasst, die mindestens zwei Segel (3) aufweist, die an einem Stützelement (2) befestigt sind, wobei die Segel einen freien Rand (5) aufweisen, der sich zwischen einer ersten Position, in der die Klappe die erste Form annimmt, und einer zweiten Position, in der die Klappe die zweite Form annimmt, bewegen kann, wobei das Verfahren das Folgende umfasst:
o Bereitstellen eines einzelnen Textilmaterialstücks, das durch Weben von Kett- und Schussfäden zu einem nahtlosen röhrenförmigen Gewebe mit mindestens einem stabilisierten Rand hergestellt wird, und
o Formen des Textilmaterialstücks zu einer Segelanordnung mit einer die Segel bildenden Innenschicht, wobei der stabilisierte Rand den freien Rand bildet, und mit einer das Stützelement bildenden Außenschicht.

2. Verfahren nach Anspruch 1, ferner umfassend das Befestigen der Segelanordnung an einem Stent.

3. Verfahren nach Anspruch 1 oder 2, wobei das einzelne Textilmaterialstück durch Schneiden eines kontinuierlichen nahtlosen röhrenförmigen Gewebes in Stücke einer gewünschten Länge und durch Stabilisieren mindestens einer der resultierenden Schneidkanten hergestellt wird.

4. Verfahren nach einem der Ansprüche 1-3, wobei die Kett- und Schussfäden thermoplastische Polymerfasern sind und der den freien Rand bildende stabilisierte Rand durch Schmelzverbindung hergestellt wird.

5. Verfahren nach einem der Ansprüche 1-4, wobei das einzelne Textilmaterialstück in einem diskontinuierlichem Verfahren hergestellt wird und der den freien Rand bildende stabilisierte Rand als Webkante gewebt wird.

6. Verfahren nach einem der Ansprüche 1-5, wobei die Prothesenklappe zwei oder drei Segel aufweist.

7. Verfahren nach einem der Ansprüche 1-6, wobei der freie Rand eines Segels eine überschüssige Länge bezüglich der zum Schließen der Klappe benötigten Mindestlänge von mindestens 5% aufweist.

8. Verfahren nach einem der Ansprüche 1-7, wobei das einzelne Textilmaterialstück durch Weben von Kett- und Schussfäden zu einem nahtlosen röhrenförmigen Einkanal-Gewebe hergestellt wird und das Bilden des Textilmaterials zu einer röhrenförmigen Segelanordnung das teilweise Umstülpen des Textilmaterialstücks zur Bildung einer Röhre in einer Röhre umfasst.

9. Verfahren nach einem der Ansprüche 1-7, wobei das einzelne Textilmaterialstück durch Weben von Kett- und Schussfäden zu einem mehrschichtigen röhrenförmigen Textilmaterial, das drei oder mehr Kanäle umfasst, hergestellt wird und das Bilden dieses Textilmaterials zu einer röhrenförmigen Segelanordnung das Umstülpen des Textilmaterialstücks umfasst.

10. Verfahren nach einem der Ansprüche 1-9, wobei das Textilmaterial mit einem Leinwandbindungs-, Körperbindungs- oder Würfelbindungsmuster oder durch eine Kombination verschiedener Webmuster hergestellt wird.

11. Verfahren nach einem der Ansprüche 1-10, wobei das Textilmaterial eine oder mehrere Schichten mit einer einzelnen Schichtdicke von 20-200 µm enthält.

12. Verfahren nach einem der Ansprüche 1-11, wobei Kett- und Schussfäden mindestens 80 Massen-% einer Art von Monofilament- oder Multifilamentgarn umfassen.

13. Verfahren nach einem der Ansprüche 1-12, wobei die Kett- und Schussfäden eine lineare Dichte von weniger als 120 dtex aufweisen.

14. Verfahren nach einem der Ansprüche 1-13, wobei die Kett- und Schussfäden Garn aus einem thermoplastischen Polymer umfassen.

15. Verfahren nach Anspruch 14, wobei die Kett- und Schussfäden Garn aus ultrahochmolekularem Polyethylen (UHMWPE) umfassen.

16. Segelanordnung für eine Prothesenklappe, die mit dem Verfahren nach einem der Ansprüche 1- 15 erhältlich ist, wobei die Segelanordnung mindestens zwei Segel aufweist, die an einem Stützelement befestigt sind, wobei die Segel einen freien Rand aufweisen, der sich zwischen einer ersten Position und einer zweiten Position bewegen kann, wobei die Segelanordnung
o aus einem einzelnen Stück eines nahtlosen röhrenförmigen Gewebes, das aus Kett- und Schussfäden besteht und mindestens einen stabilisierten Rand aufweist, hergestellt wird, und
o eine die Segel bildende Innenschicht, wobei der stabilisierte Rand den freien Rand bildet, und eine das Stützelement bildende Außenschicht aufweist.

17. Prothesenklappe, die mit dem Verfahren nach einem der Ansprüche 1-15 erhältlich ist, wobei die Klappe eine erste Form, in der die Klappe geöffnet ist, und eine zweite Form, in der die Klappe geschlossen ist, annehmen kann, und eine Segelanordnung umfasst, die mindestens zwei Segel aufweist, die an einem Stützelement befestigt sind, wobei die Segel einen freien Rand aufweisen, der sich zwischen einer ersten Position, in der die Klappe die erste Form annimmt, und einer zweiten Position, in der die Klappe die zweite Form annimmt, bewegen kann, und wobei die Segelanordnung:
o aus einem einzelnen Stück eines nahtlosen röhrenförmigen Gewebes, das aus Kett- und Schussfäden besteht und mindestens einen stabilisierten Rand aufweist, hergestellt wird, und
o eine die Segel bildende Innenschicht, wobei der stabilisierte Rand den freien Rand bildet, und eine das Stützelement bildende Außenschicht aufweist.

18. Prothesenklappe nach Anspruch 17, ferner umfassend einen Stent, der an der Segelanordnung befestigt ist.

19. Prothesenklappe nach Anspruch 17 oder 18, wobei die Klappe zwei oder drei Segel umfasst, wobei jedes Segel als eine Verschlussfläche für das andere bzw. die anderen Segel wirkt.

## Revendications

1. Procédé de fabrication d'une valvule prothétique (400) qui peut prendre une première forme dans laquelle la valvule est ouverte et une seconde forme dans laquelle la valvule est fermée, la valvule comprenant un ensemble de feuillets comportant au moins deux feuillets (3) fixés à un élément de support (2), les feuillets possédant une marge libre (5) qui peut se déplacer entre une première position dans laquelle la valvule prend la première forme et une seconde position dans laquelle la valvule prend la seconde forme, le procédé consistant à :
∘ fournir une pièce unique de tissu, réalisée en tissant des fils de chaîne et de trame en un tissu tissé tubulaire sans couture possédant au moins un bord stabilisé, et
∘ former la pièce de tissu en un ensemble de feuillets possédant une couche interne formant les feuillets, le bord stabilisé formant la marge libre, et une couche externe formant l'élément de support.

2. Procédé selon la revendication 1, consistant en outre à fixer l'ensemble de feuillets à un stent.

3. Procédé selon la revendication 1 ou 2, dans lequel la pièce unique de tissu est réalisée en coupant un tissu tissé tubulaire sans couture continu en morceaux de longueur souhaitée, et en stabilisant au moins un des bords coupés ainsi obtenus.

4. Procédé selon l'une quelconque des revendications 1 à 3, dans lequel les fils de chaîne et de trame sont des fibres de polymère thermoplastique et le bord stabilisé formant la marge libre est réalisé par fusion.

5. Procédé selon l'une quelconque des revendications 1 à 4, dans lequel la pièce unique de tissu est fabriquée dans un procédé discontinu et le bord stabilisé formant la marge libre est tissé sous la forme d'une lisière.

6. Procédé selon l'une quelconque des revendications 1 à 5, dans lequel la valvule prothétique possède deux ou trois feuillets.

7. Procédé selon l'une quelconque des revendications 1 à 6, dans lequel la marge libre d'un feuillet présente une longueur excédentaire par rapport à la longueur minimum requise pour fermer la valvule d'au moins 5 %.

8. Procédé selon l'une quelconque des revendications 1 à 7, dans lequel la pièce unique de tissu est fabriquée en tissant des fils de chaîne et de trame en un tissu tissé tubulaire sans couture monocanal, et la formation du tissu en un ensemble de feuillets tubulaire consiste à retourner partiellement la pièce de tissu dans le but de former un tube dans un tube.

9. Procédé selon l'une quelconque des revendications 1 à 7, dans lequel la pièce unique de tissu est fabriquée en tissant des fils de chaîne et de trame en un tissu tubulaire multicouche comprenant trois canaux ou plus, et la formation d'un tel tissu en un ensemble de feuillets tubulaire consiste à retourner la pièce de tissu tubulaire.

10. Procédé selon l'une quelconque des revendications 1 à 9, dans lequel le tissu est fabriqué avec un motif d'armure toile, d'armure sergé ou d'armure natté, ou par une combinaison de différents motifs de tissage.

11. Procédé selon l'une quelconque des revendications 1 à 10, dans lequel le tissu contient une ou plusieurs couches ayant une épaisseur de couche unique de 20 à 200 µm.

12. Procédé selon l'une quelconque des revendications 1 à 11, dans lequel les fils de chaîne et de trame comprennent au moins 80 % en masse d'un type de fil monofilament ou multifilament.

13. Procédé selon l'une quelconque des revendications 1 à 12, dans lequel les fils de chaîne et de trame ont une densité linéaire inférieure à 120 dtex.

14. Procédé selon l'une quelconque des revendications 1 à 13, dans lequel les fils de chaîne et de trame comprennent un fil de polymère thermoplastique.

15. Procédé selon la revendication 14, dans lequel les fils de chaîne et de trame comprennent un fil de polyéthylène de masse moléculaire très élevée (UHMWPE).

16. Ensemble de feuillets pour une valvule prothétique telle qu'elle peut être obtenue par le procédé selon l'une quelconque des revendications 1 à 15, l'ensemble de feuillets comportant au moins deux feuillets fixés à un élément de support, les feuillets possédant une marge libre qui peut se déplacer entre une première position et une seconde position, l'ensemble de feuillets
∘ étant fabriqué à partir d'une pièce unique de tissu tissé tubulaire sans couture réalisée à partir de fils de chaîne et de trame et possédant au moins un bord stabilisé, et
∘ comportant une couche interne formant les feuillets, le bord stabilisé formant la marge libre, et une couche externe formant l'élément de support.

17. Valvule prothétique telle qu'elle peut être obtenue par le procédé selon l'une quelconque des revendications 1 à 15, laquelle valvule peut prendre une première forme dans laquelle la valvule est ouverte et une seconde forme dans laquelle la valvule est fermée, et qui comprend un ensemble de feuillets comportant au moins deux feuillets fixés à un élément de support, les feuillets possédant une marge libre qui peut se déplacer entre une première position dans laquelle la valvule prend la première forme et une seconde position dans laquelle la valvule prend la seconde forme, et dans laquelle l'ensemble de feuillets
∘ est fabriqué à partir d'une pièce unique de tissu tissé tubulaire sans couture, réalisée à partir de fils de chaîne et de trame et possédant au moins un bord stabilisé, et
∘ comporte une couche interne formant les feuillets, le bord stabilisé formant la marge libre, et une couche externe formant l'élément de support.

18. Valvule prothétique selon la revendication 17, comprenant en outre un stent fixé à l'ensemble de feuillets.

19. Valvule prothétique selon la revendication 17 ou 18, la valvule comprenant deux ou trois feuillets, chaque feuillet faisant office de surface de fermeture pour le ou les autres feuillets.
